# EUROPEAN PATENT SPECIFICATION

(11) **EP 2 046 841 B1**
(45) Date of publication and mention of the grant of the patent: **03.03.2010**
(21) Application number: 07787951.8
(22) Date of filing: 26.07.2007
(51) Int. Cl.: C08F 4/622, C08F 4/70, C08F 10/02, C07C 251/70

(54) **POLYMERISATION CATALYST SYSTEM BASED ON OXIME-ETHER LIGANDS**
POLYMERISATIONSKATALYSATORSYSTEM AUF BASIS VON OXIMETHER-LIGANDEN
SYSTÈME CATALYTIQUE DE POLYMÉRISATION BASÉ SUR DES LIGANDS OXIME-ÉTHER

(30) Priority: 03.08.2006 EP 06291272
(43) Date of publication of application: 15.04.2009
(73) Proprietor: Total Petrochemicals Research Feluy, 7181 Seneffe (Feluy) (BE); Centre National de la Recherche Scientifique (CNRS), 75016 Paris (FR)
(72) Inventor: BOULANGER, Loïse, F-54500 Vandoeuvre Les Nancy (FR); LAVASTRE, Olivier, 35490 Gahard (FR)
(86) International application number: PCT/EP2007/057734
(87) International publication number: WO 2008/015160

(56) References cited:
- JONES, RAYMOND C. F. ET AL: "A chiral imidazoline nitrone; a cycloaddition route to imidazoisoxazoles and pyrroloimidazoles" SYNLETT , (7), 967-970 CODEN: SYNLES; ISSN: 0936-5214, 2000, XP002411995
- FISCHER, H. P. ET AL: "Preparation and determination of configuration of syn- and anti-.alpha.-aminoketoxime derivatives" HELVETICA CHIMICA ACTA , 45, 2528-38 CODEN: HCACAV; ISSN: 0018-019X, 1962, XP009076377
- FISCHER, H. P. ET AL: "Fragmentation of .alpha.-amino ketoximes. Beckmann reactions of the second type. I. Fragmentation reactions. 4" HELVETICA CHIMICA ACTA , 45, 2539-53 CODEN: HCACAV; ISSN: 0018-019X, 1962, XP009076378

## Description

This invention relates to the field of oxime-ether ligands and their use in catalyst system for the polymerisation and oligomerisation of ethylene and alpha-olefins.

There exists a multitude of catalyst systems available for polymerising or oligomerising ethylene and alpha-olefins, but there is a growing need for finding new systems capable to tailor polymers with very specific properties. More and more post-metallocene catalyst components based on early or late transition metals from Groups 3 to 10 of the Periodic Table have recently been investigated such as for example those disclosed in Gibson and al. review (Gibson, V.C.; Spitzmesser, S.K., Chem. Rev. 2003, 103, p. 283). But there is still a need to improve either the specificities or the performances of these systems.

It is an aim of the present invention to prepare a polymerisation catalyst system based on oxime-ether ligands.

It is also an aim of the present invention to use oxime-ether ligand-based catalyst system for the homo- or co-polymerisation of ethylene and alpha-olefins.

Accordingly, the present invention discloses oxime-ether ligands of general formula I 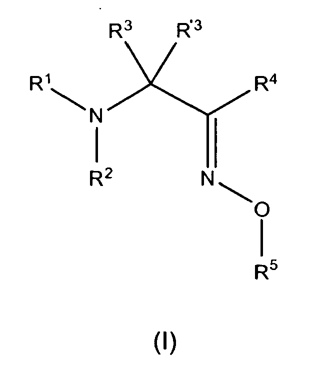 wherein wherein R¹, R², R³, R'³ and R⁴ are each independently selected from hydrogen or alkyl group having from 1 to 20 carbon atoms, or aryl group having from 3 to 18 carbon atoms or heterocycles or wherein two neighbouring Rⁱ can be linked together to form a ring and
wherein R⁵ is a alkyl, benzyl or phenyl compound of formula 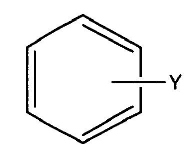 wherein there is at least one substituent Y on the phenyl group, and each Y is the same or different and at least one Y is an electron attracting group.

The other Y substituents, if present, can be steric substituents such as for example alkyl or aryl groups.

Preferably, the electron attracting group Y is NO₂ or CN.

Among the preferred embodiments according to the present invention, R¹ and R² can each be independently selected from isopropyl, n-butyl, benzyl, cyclohexyl, pyridyl, méthylpyridine, thiényl, thényl, furyl, furfuryl, phenyl, mesityl. R³ and R'³ are preferably hydrogen and R⁴ is preferably an alkyl group having from 1 to 6 carbon atoms, more preferably methyl.

The invention also discloses a process for preparing an oxime-ether ligand that comprises the steps of:
a) providing an oxime ligand of formula II 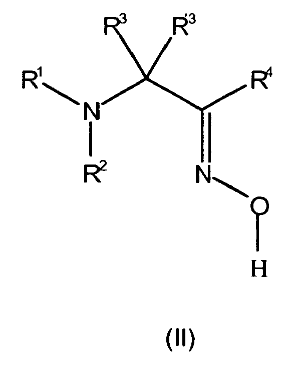 wherein R¹, R², R³, R'³ and R⁴ are each independently selected from H or alkyl groups having from 1 to 20 carbon atoms or aryl groups having from 3 to 18 carbon atoms or functional groups such as heterocycles or two neighbouring Rⁱ can be linked together to form a ring.
b) deprotonating the OH group of the oxime ligand in the presence of a base;
c) in the presence of a polar solvent; and
d) optionally in the presence of a crown ether able to trap the cation from the base of step b).
e) reacting the anion obtained from steps b), c) and d) with R⁵-X wherein X is a halogen, and R⁵ is alkyl or aryl, preferably R⁵-X is BnBr where Bn is benzyl, or a phenyl group carrying a fluor substitutent and at least one other substituent Y that is an electron attracting group.

The preparation of the ligand can typically be represented by the following scheme. 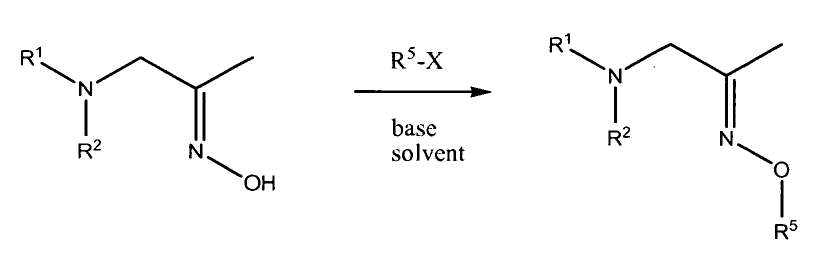 wherein R¹, R² and R⁵ are as described hereabove.

The catalyst component is then prepared by complexing the ligand with a metallic precursor MXᵥ in a ligand to metal ratio of from 1/1 to 2/1. The metallic precursor and the ligand are placed in a solvent and they are allowed to react under stirring for a period of time of from 2 to 10 hours at a temperature of from 10 to 80 °C preferably at room temperature (about 25 °C).

Metal M is selected from groups 6 to 10 of the Periodic Table. Preferably, it is Cr, Fe, Co, Ni, Pd, more preferably it is nickel. X is halogen and v is the valence of M.

The solvent is polar or apolar. Preferably it is tetrahydrofuran (THF).

An active catalyst system is then prepared by adding an activating agent having an ionising action.

Any activating agent having an ionising action known in the art may be used for activating the monooxime catalyst component. For example, it can be selected from aluminium-containing or boron-containing compounds. The aluminium-containing compounds comprise aluminoxane and/or alkyl aluminium.

The aluminoxanes are preferred and may comprise oligomeric linear and/or cyclic alkyl aluminoxanes represented by the formula: 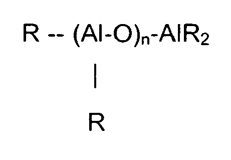 for oligomeric, linear aluminoxanes and 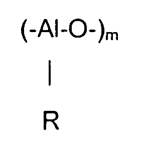 for oligomeric, cyclic aluminoxane,
wherein n is 1-40, preferably 10-20, m is 3-40, preferably 3-20 and R is a C₁-C₈ alkyl group and preferably methyl.

Suitable boron-containing activating agents that can be used comprise a triphenylcarbenium boronate such as tetrakis-pentafluorophenyl-borato-triphenylcarbenium as described in EP-A-0427696, or those of the general formula [L'-H] + [B Ar₁ Ar₂ X₃ X₄]- as described in EP-A-0277004 (page 6, line 30 to page 7, line 7).

The preferred activating agent is aluminoxane. The amount of aluminoxane necessary to activate the catalyst component is selected to have a Al/M ratio of from 100 to 3000, preferably about 1000.

The catalyst system can also be supported. The support if present can be a porous mineral oxide, advantageously selected from silica, alumina and mixtures thereof. Preferably it is silica.

The present invention also discloses a method for oligomerising and for homo- or copolymerising ethylene and alpha-olefins that comprises the steps of:
a) injecting the active catalyst system into the reactor;
b) injecting the monomer and optional comonomer into the reactor;
c) optionally injecting a scavenger;
d) maintaining under polymerising conditions;
e) retrieving the oligomers and polymers.

The optional scavenger is preferably aluminium alkyl, more preferably triisobutyl aluminium (TIBAL);

The polymerisation and oligomerisation method is not particularly limited and it can be carried out at a temperature of from 20 to 85°C and under a pressure of from 0.5 to 50 bars. Preferably, the pressure is of at least 15 bars, more preferably of at least 20 bars.

The preferred monomers and comonomers are selected from ethylene, propylene and hexene.

### List of figures.

Figure 1 represents the molecular weight distribution of the polymer obtained with a catalyst system based on metallic complex C1.

Figure 2 represents the molecular weight distribution of the polymer obtained with a catalyst system based on metallic complex C2.

Figure 3 represents the molecular weight distribution of the polymer obtained with a catalyst system based on metallic complex C3.

Figure 4 represents the molecular weight distribution of the polymer obtained with a catalyst system based on metallic complex C4.

### Examples.

### 1. Synthesis of ligand L1 - benzyl-furan-2-ylmethyl-amino)-propan-2-one 0-benzyl-oxime.

In a balloon containing 1.75 mmol of oxime ligand, 25 mL of dimethylformamide (DMF) were added, then 1.75 mmol of NaH. The mixture was kept under stirring for a period of time of one hour. 1.75 mmol of BnBr were then added and stirring was maintained for a period of time of 20 hours. The solvent was vaporised and the mixture was purified by silica gel chromatography to obtain the resulting oxime-ether with a yield of 91 % as a pale yellow oil.

The following scheme was used:

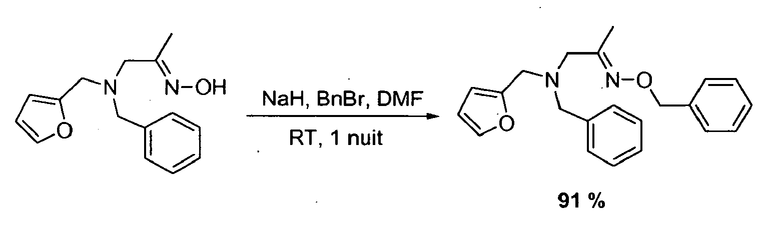

The compound was characterised by NMR.
**RMN ¹H** (300 MHz, CDCl₃) : 7.28-7.40 (m, 11H), 6.34 (m, 1H), 6.17 (m, 1H), 5.13 (s, 2H), 3.60 (s, 2H), 3.58 (s, 2H), 3.13 (s, 2H), 1.96 (s, 3H);
**RMN** ¹³C (75 MHz, CDCl₃) : 157.1, 152.2, 142.0, 138.9, 138.3, 128.9, 128.3, 128.2, 127.8, 127.6, 127.0, 110.1, 108.9, 75.4, 57.4, 57.3, 49.5, 13.1;
**EIMS** *m*/*z* [M-CH₂C₄H₃O]⁺ 267.1498, calcd for C₁₇H₁₉N₂O 267.1497; Anal. Calcd C, 75.83; H, 6.94; N, 8.04. Found: C, 75.83; H, 6.89; N, 8.02

### 2. Synthesis of aryl-substituted oxime-ether ligands.

The introduction of a simple phenyl group was impossible for lack of reactivity of fluorobenzene. Fluorinated derivatives activated with electro-attracting substituents were selected.
The general procedure was the same as that used for the preparation of O-benzyle oxime-ether ligands and the following scheme was used.

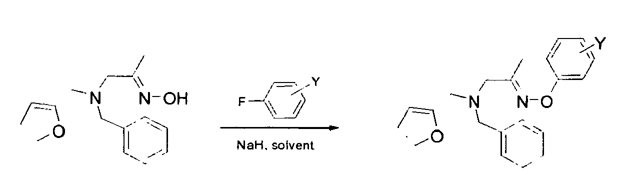

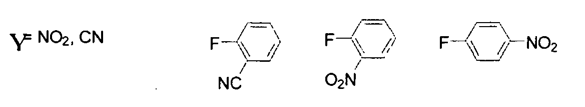

Specific amounts of reactants and solvents were selected according to each substituted fluorobenzene.

### Ligand L2 - (1-benzyl-furan-2-ylmethyl-amino)-propan-2-one 0-(2-nitro-phenyl)-oxime.

For Y = NO₂

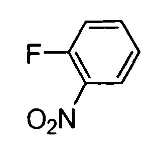

1.5 equivalents of fluorinated derivative substrate were used for 1 equivalent of NaH and 1 equivalent of oxime. The solvent was tetrahydrofurane (THF) 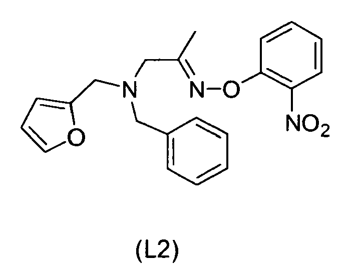 was obtained with a yield of 87 % as a yellow oil.

The compound was characterised by NMR.
**RMN ¹H** (300 MHz, CDCl₃) : 7.97 (dd, *J*¹ = 1.5 Hz, *J*₂ = 8.3 Hz, 1H), 7.74 (dd, *J*₁ = 1.1 Hz, *J*₂ = 8.3 Hz, 1H), 7.57 (td, *J*₁ = 1.5 Hz, *J*₂ = 7.9 Hz, 1H), 7.26-7.42 (m, 7H), 7.06 (td, *J*₁ = 1.5 Hz, *J*₂ = 7.9 Hz, 1H), 6.35 (dd, *J*₁ = 1.9 Hz, *J*₂ = 3.4 Hz, 1H), 6.23 (d, *J* = 3.0 Hz, 1H), 3.67 (s, 2H), 3.64 (s, 2H), 3.27 (s, 2H), 2.17 (s, 3H);
**RMN** ¹³C (75 MHz, CDCl₃) : 163.6, 153.1, 151.9, 142.2, 138.5, 137.3, 134.5, 129.0, 128.9, 128.8, 128.4, 127.3, 125.5, 121.2, 117.1, 110.2, 109.1, 57.8, 56.7, 49.8, 14.3;
**EIMS** *m*/*z* [M-CH₂C₄H₃O]⁺ 298.1197, calcd for C₁₆H₁₆N₃O₃ 298.1192; **Anal**. Calcd C, 66.48; H, 5.58; N, 11.07. Found: C, 67.22; H, 6.04; N, 10.32.
Or

### Ligand L3 - 1-(benzyl-furan-2-ylmethyl-amino)-propan-2-one 0-(4-nitro-phenyl)-oxime.

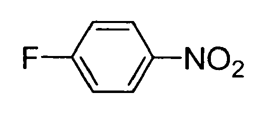

1 equivalent of fluorinated derivative substrate was used for 1 equivalent of NaH and 1 equivalent of oxime. The solvent was DMF. 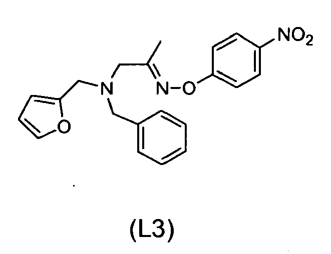 was obtained with a yield of 60 % as an orange oil.
NMR results were as follows.

**RMN ¹H** (300 MHz, CDCl₃) : 8. 21 (m, 2H), 7.25-7.39 (m, 8H), 6.36 (dd, *J*₁ = 1.9 Hz, *J*₂ = 3.0 Hz, 1H), 6.24 (d, *J* = 3.0 Hz, 1H), 3.68 (s, 2H), 3.66 (s, 2H), 3.29 (s, 2H), 2.11 (s, 3H);
**RMN ¹³C** (75 MHz, CDCl₃) : 164.1, 162.8, 151.8, 142.2, 138.5, 128.9, 128.4, 127.3, 125.7,114.3, 110.2, 109.1, 57.8, 56.9, 49.9, 13.8;
**EIMS** *m*/*z* [M-CH₂Ph]⁺ 288.0991, calcd for C₁₄H₁₄N₃O₄ 288.0984; **Anal**. Calcd C, 66.48; H, 5.58; N, 11.07. Found: C, 66.97; H, 5.61; N, 11.03.

### Ligand L4 - 2-[2-(benzyl-furan-2-ylmethyl-amino)-1-methyl-ethylideneaminooxy]-benzonitrile

For Y=CN

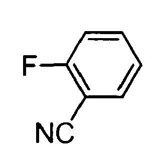

1.5 equivalents of fluorinated derivative substrate were used for 1 equivalent of NaH and 1 equivalent of oxime. The solvent was DMF. 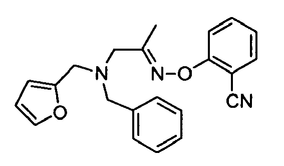 was obtained with a yield of 68 % as a pale yellow oil.

NMR results were as follows.
**RMN ¹H** (300 MHz, CDCl₃) : 7.52-7.56 (m, 3H), 7.26-7.42 (m, 7H), 7.01-7.06 (m, 1H), 6.35 (dd, *J*₁ = 1.9 Hz, *J*₂ = 3.4 Hz, 1H), 6.23 (d, *J* = 3.0 Hz, 1H), 3.68 (s, 2H), 3.65 (s, 2H), 3.28 (s, 2H), 2.17 (s, 3H);
**RMN ¹³C** (75MHz, CDCl₃) 163.1, 161.0, 151.9, 142.2, 138.5, 134.3, 133.0, 128.9, 128.4, 127.3, 121.8, 116.0, 114.9, 110.2, 109.1, 99.4, 57.8, 56.7, 49.8, 13.9;
**EIMS** *m*/*z* [M]⁺ 359.1638, calcd for C₂₂H₂₁N₃O₂ 359.1634; **Anal**. Calcd C, 73.52; H, 5.89; N, 11.69. Found: C, 73.47; H, 5.91; N, 11.66.

### Synthesis of chromium complexes.

In a glove box, CrCl₂ was introduced in a Schlenk and a solution of the ligand in THF was added: the metal concentration was of 3.10⁻² mol/L. The complexation reaction was carried out for a period of time of 6 hours under stirring and then THF was eliminated under vacuum. The solid residue was washed three times with ether in order to eliminate all residual ligand and then dried under vacuum.

The amounts of ligand and metallic complex are summarised in Table I.

**TABLE I.**

| Catalyst component | Ligand used | Proportions metal/ligand | Complex colour |
|---|---|---|---|
| C1 | L1 | 1/1.2 | Green |
| C2 | L2 | 1/1 | Brown |
| C3 | L3 | 1/1.2 | Brown |
| C4 | L4 | 1/1.2 | Green |

### Polymerisation of ethylene.

All catalyst components were activated with methylaluminoxane (MAO) with a ratio Al/Cr of 1000, the solvent was toluene, the polymerisation temperature was of 35 °C and the ethylene pressure was of 15 bars.

The metallic complex was added to a MAO solution (30% in toluene, 730 equ.), and the mixture was stirred for a period of time of 5 to 10 minutes. In the reactor under inert atmosphere were successively added 50 mL of toluene, a scavenger solution consisting of MAO (30% , 270 equ.), completed to 5 mL with toluene and the solution of activated metallic complex. The temperature was increased to its target value of 35 °C and the ethylene pressure was increased to 15 bars. These conditions were maintained during the reaction time of one hour.

After degassing, the oligomers and polymers were retrieved. The polymer was washed with MeOH/HCl 5% then with MeOH and finally with acetone. It was then dried under vacuum overnight. The results are reported in Table II.

**TABLE II.**

| Metal complex | Cata µmol | Activity KgPE/molCr/h | Consumption KgC₂H₄/molCr/h | Polymer/oligomer |
|---|---|---|---|---|
| C1 | 8.6 | 31 | 2187 | 1/70 |
| C2 | 9.4 | 79 | 1960 | 1/25 |
| C3 | 9.6 | 32 | 1790 | 1/56 |
| C4 | 10.2 | 100 | 1356 | 1/14 |

The activity is measured with respect to the polymer production.
The ethylene consumption curves showed very little decrease.

It can be seen that the catalytic systems of the present invention produce simultaneously oligomers and polymers, with a predominance for oligomers. Several catalytic species are thus probably simultaneously present.

Ethylene consumption for all complexes was high. In ethylene polymerisation, ligand C4, functionalised with a cyano group, was the most active and gave the highest polymer/oligomer ratio.

The oligomer analysis carried out by gas chromatography showed predominantly the formation of alpha-oligomers, with a proportion of up to 95 %. In addition, all systems had a similar Shultz-Flory type oligomer distribution up to C₂₄. The C₆/(C₄+C₆) ratio was in the range 0.57 to 0.60.

The polymers were studied by Gel permeation Chromatographt (GPC) and by Differential Scanning Calorimetry (DSC). The polidispersity index PI is the ratio Mw/Mn of the weight average molecular weight Mw over the number average molecular weight Mn. Their properties are summarised in Table III.

**TABLE III.**

| Metal complex | Mn kD | Mw kD | PI | Tm °C |
|---|---|---|---|---|
| C1 | 8177 | 301100 | 36.8 | 125.0 |
| C2 | 8770 | 558768 | 63.7 | 126.9 |
| C3 | 10464 | 646838 | 61.8 | 114.7 |
| C4 | 5599 | 148652 | 26.6 | 127.3 |

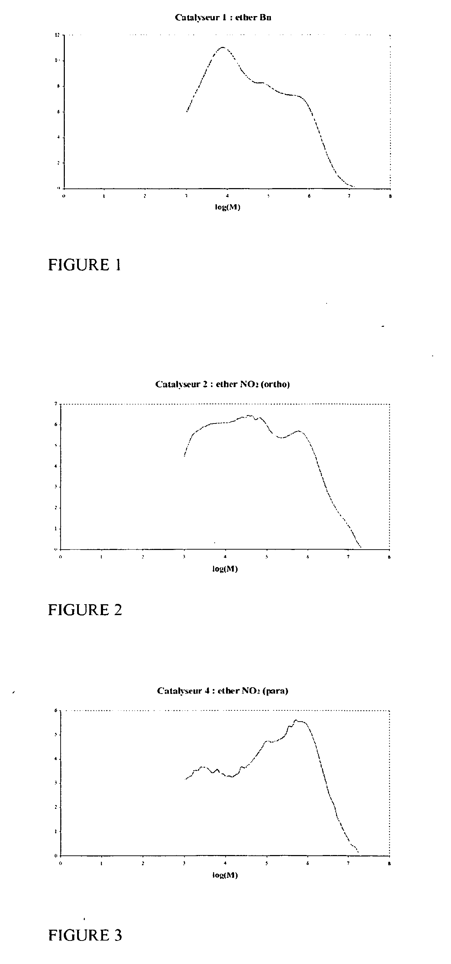

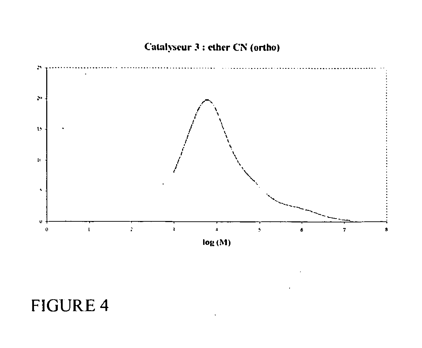

The molecular weight distributions of the polymers produced with catalyst systems based on C1 to C4 are represented respectively in Figures 1 to 4. it can be seen that the polymers prepared with C1 to C3 have a large polydispersity index and a multimodal molecular weight distribution. The polymer formed with complex C4 has a monomodal molecular weight distribution.

The influence of polymerisation temperature and ethylene pressure have been studied for the catalyst systems based on metallic complexes C1 and C4. The results are displayed in Table IV.

**TABLE IV.**

| Metal complex | T °C | P bar | Cr µmol | Activity KgPE/molCr/h | Consump. KgC2/molCr/h | Poly/oligo |
|---|---|---|---|---|---|---|
| C1 | 35 | 15 | 8.6 | 31 | 2187 | 1/70 |
| | 60 | 15 | 10.6 | 20 | 2309 | 1/115 |
| | 35 | 24 | 5.7 | 101 | 2333 | 1/23 |
| C4 | 35 | 15 | 10.3 | 100 | 1356 | 1/14 |
| | 60 | 15 | 9.3 | 37 | 1914 | 1/52 |
| | 35 | 24 | 9.3 | 299 | 2784 | 1/9 |

It can be seen that for the two catalyst systems studied, an increase in temperature increased the ethylene consumption but reduced the activity in polymer production and the ratio of polymer to oligomer. It was also observed that the stability of the catalyst system decreased with increasing temperature.

Increasing the pressure had a positive impact on all counts: it increased the activity, the ethylene consumption, the polymer to oligomer ratio and the stability.

Preferably, the polymerisation of ethylene should be carried at an ethylene pressure of at least 15 bars, more preferably of at least 20 bars.

## Claims

1. A process for preparing a catalyst component by complexing an oxime-ether ligang of formula I 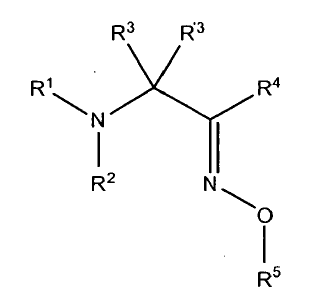 with metallic precursor MXᵥ,
wherein R¹, R², R³, R'³ and R⁴ are each independently selected from hydrogen or alkyl group having from 1 to 20 carbon atoms, or aryl group having from 3 to 18 carbon atoms or heterocycles or wherein two neighbouring Rⁱ can be linked together to form a ring and
wherein R⁵ is a alkyl, benzyl or phenyl compound of formula 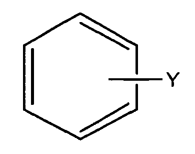 wherein there is at least one substituent Y on the phenyl group, and each additional Y is the same or different and the at least one Y is an electron attracting group,
wherein M is a metal Group 6 to 10 of the Periodic Table,
wherein X is halogen and v is the valence of M, and
wherein the ratio ligand/metal of 1:1 to 2:1.

2. The process of claim 1 wherein the additional Y substituents are steric substituents selected from alkyl and aryl goups having up to 12 carbon atoms.

3. The process of claim 1 or claim 2 wherein R¹ and R² are independently selected from isopropyl, n-butyl, benzyl, cyclohexyl, pyridyl, méthylpyridine, thiényl, thényl, furyl, furfuryl, phenyl or mesityl.

4. The process of any one of claims 1 to 3 wherein R³ and R'³ are hydrogen.

5. The process of any one of the preceding claims wherein R⁴ is an alkyl group having from 1 to 6 carbon atoms, preferably methyl.

6. The process of any one of the preceding claims wherein R⁵ is benzyl.

7. The process of any one of the preceding claims wherein R⁵ is a phenyl group carrying a fluor substitutent and at least one other substituent Y that is an electron attracting group.

8. The process of claim 7 wherein the at least one other substituent Y is an electron attracting group selected from CN or NO₂.

9. The process of any one of the preceding claims wherein metal M is Cr, Fe, Co, Ni, Pd.

10. A metallic component obtainable by the process of any one of claims 1 to 9.

11. An active catalyst system comprising the metallic component of claim 10 and an activating agent having an ionising action.

12. The active catalyst system of claim 11 wherein the activating agent is methylaluminoxane.

13. A process for oligomerising and for homopolymerising ethylene and alpha-olefins that comprises the steps of:
a) injecting the active catalyst system of claim 11 or claim 12 into the reactor;
b) injecting the monomer and optional comonomer into the reactor;
c) optionally injecting a scavenger;
d) maintaining under polymerising conditions;
e) retrieving the oligomers and polymers.

14. The process of claim 13 wherein the monomer is ethylene, propylene or 1-hexene, preferably ethylene.

15. The process of claim 13 or claim 14 wherein the monomer is ethylene and the ethylene pressure is of at least 20 bars.

## Patentansprüche

1. Verfahren zur Herstellung einer Katalysatorkomponente durch Komplexierung eines Oximether-Liganden der Formel I 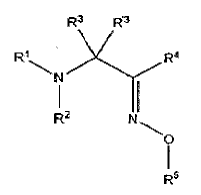 mit metallischem Präkursor MXᵥ,
wobei R¹, R², R³, R'³ und R⁴ jedes unabhängig ausgewählt sind aus Wasserstoff oder Alkylgruppe mit 1 bis 20 Kohlenstoffatomen, oder Arylgruppe mit 3 bis 18 Kohlenstoffatomen, oder Heterocyclen, oder wobei zwei benachbarte Rⁱ miteinander verknüpft werden können, um einen Ring zu bilden, und
wobei R⁵ eine Alkyl-, Benzyl- oder Phenylverbindung der Formel 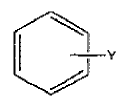 ist, wobei mindestens ein Substituent Y an der Phenylgruppe vorkommt, und jedes zusätzliche Y das gleiche oder verschieden ist und das mindestens eine Y eine elektronenziehende Gruppe ist,
wobei M ein Metall der Gruppe 6 bis 10 der Tabelle des Periodensystems ist,
wobei X Halogen ist und v die Valenz von M ist, und
wobei das Verhältnis von Ligand zu Metall 1:1 bis 2:1 beträgt.

2. Verfahren nach Anspruch 1, wobei die zusätzlichen Y-Substituenten sterische Substituenten sind, ausgewählt aus Alkyl- und Arylgruppen mit bis zu 12 Kohlenstoffatomen.

3. Verfahren nach Anspruch 1 oder Anspruch 2, wobei R¹ und R² unabhängig aus Isopropyl, n-Butyl, Benzyl, Cyclohexyl, Pyridyl, Methylpyridin, Thienyl, Thenyl, Furyl, Furfuryl, Phenyl oder Mesityl ausgewählt sind.

4. Verfahren nach einem der Ansprüche 1 bis 3, wobei R³ und R'³ Wasserstoff sind.

5. Verfahren nach einem der vorgenannten Ansprüche, wobei R⁴ eine Alkylgruppe mit 1 bis 6 Kohlenstoffatomen ist, bevorzugt Methyl.

6. Verfahren nach einem der vorgenannten Ansprüche, wobei R⁵ Benzyl ist.

7. Verfahren nach einem der vorgenannten Ansprüche, wobei R⁵ eine Phenylgruppe ist, die einen Fluorsubstituenten und mindestens einen anderen Substituenten Y, der eine elektronenziehende Gruppe ist, trägt.

8. Verfahren nach Anspruch 7, wobei der mindestens eine andere Substituent Y eine aus CN oder NO₂ ausgewählte elektronenziehende Gruppe ist.

9. Verfahren nach einem der vorgenannten Ansprüche, wobei das Metall M Cr, Fe, Co, Ni, Pd ist.

10. Metallische Komponente, erhaltbar durch das Verfahren nach einem der Ansprüche 1 bis 9.

11. Aktives Katalysatorsystem, das die metallische Komponente von Anspruch 10 und ein Aktivierungsmittel mit ionisierender Wirkung umfasst.

12. Aktives Katalysatorsystem nach Anspruch 11, wobei das Aktivierungsmittel Methylaluminoxan ist.

13. Verfahren zum Oligomerisieren und zum Homopolymerisieren von Ethylen und Alpha-Olefnen, das die Schritte umfasst des:
a) Injizierens des aktiven Katalysatorsystems von Anspruch 11 oder Anspruch 12 in den Reaktor;
b) Injizierens des Monomers und optionalen Comonomers in den Reaktor;
c) optionsweisen Injizierens eines Scavengers;
d) Haltens unter Polymerisationsbedingungen;
e) Rückgewinnens der Oligomere und Polymere.

14. Verfahren nach Anspruch 13, wobei das Monomer Ethylen, Propylen oder 1-Hexen, bevorzugt Ethylen, ist.

15. Verfahren nach Anspruch 13 oder Anspruch 14, wobei das Monomer Ethylen ist und der Ethylendruck mindestens 20 bar beträgt.

## Revendications

1. Procédé pour préparer un composant faisant office de catalyseur par complexation d'un ligand oxime-éther répondant à la formule I 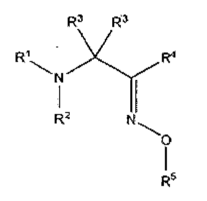 avec un précurseur métallique MXᵥ,
formule dans laquelle R¹, R², R³, R^{3'} et R⁴ sont choisis, chacun indépendamment l'un de l'autre, parmi le groupe comprenant un atome d'hydrogène ou un groupe alkyle contenant de 1 à 20 atomes de carbone ou un groupe aryle contenant de 3 à 18 atomes de carbone ou des composés hétérocycliques, ou bien dans laquelle deux radicaux R¹ voisins peuvent être liés l'un à l'autre pour former un noyau, et
dans laquelle R⁵ représente un groupe alkyle, un groupe benzyle ou un groupe phényle répondant à la formule 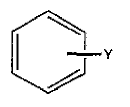 le groupe phényle possédant au moins un substituant Y et chaque substituant Y supplémentaire étant identique ou différent, ledit au moins un substituant Y représentant un groupe attirant les électrons ;
M représente un métal des Groupes 6 à 10 du tableau périodique,
X représente un atome d'halogène et v représente la valence de M, et
le rapport ligand/métal à s'élève de 1:1 à 2:1.

2. Procédé selon la revendication 1, dans lequel les substituants Y supplémentaires sont des substituants stériques choisis parmi un groupe alkyle et un groupe aryle contenant jusqu'à 12 atomes de carbone.

3. Procédé selon la revendication 1 ou 2, dans lequel R¹ et R² sont choisis, de manière indépendante, parmi un groupe isopropyle, un groupe n-butyle, un groupe benzyle, un groupe cyclohexyle, un groupe pyridyle, un groupe méthylpyridine, un groupe thiényle, un groupe thényle, un groupe furyle, un groupe furfuryle, un groupe phényle ou un groupe mésityle.

4. Procédé selon l'une quelconque des revendications 1 à 3, dans lequel R³ et R^{3'} représentent un atome d'hydrogène.

5. Procédé selon l'une quelconque des revendications précédentes, dans lequel R⁴ représente un groupe alkyle contenant de 1 à 6 atomes de carbone, de préférence un groupe méthyle.

6. Procédé selon l'une quelconque des revendications précédentes, dans lequel R⁵ représente un groupe benzyle.

7. Procédé selon l'une quelconque des revendications précédentes, dans lequel R⁵ représente un groupe phényle portant un substituant fluoro et au moins un autre substituant Y qui représente un groupe attirant les électrons.

8. Procédé selon la revendication 7, dans lequel ledit au moins autre substituant Y représente un groupe attirant les électrons, choisi parmi un groupe CN ou un groupe NO₂.

9. Procédé selon l'une quelconque des revendications précédentes, dans lequel le métal M représente Cr, Fe, Co, Ni, Pd.

10. Composant métallique que l'on peut obtenir via le procédé selon l'une quelconque des revendications 1 à 9.

11. Système de catalyseur actif comprenant le composant métallique selon la revendication 10 et un agent d'activation possédant une action ionisante.

12. Système de catalyseur actif selon la revendication 11, dans lequel l'agent d'activation est le méthylaluminoxane.

13. Procédé pour l'oligomérisation et pour l'homopolymérisation d'éthylène et d'alpha-oléfines, qui comprend les étapes consistant à :
a) injecter le système de catalyseur actif selon la revendication 11 ou 12 dans le réacteur ;
b) injecter le monomère et le comonomère facultatif dans le réacteur ;
c) injecter de manière facultative un fixateur ;
d) maintenir dans des conditions de polymérisation ;
e) retirer les oligomères et les polymères.

14. Procédé selon la revendication 13, dans lequel le monomère représente l'éthylène, le propylène ou le 1-hexène, de préférence l'éthylène.

15. Procédé selon la revendication 13 ou 14, dans lequel le monomère représente l'éthylène et la pression de l'éthylène s'élève à au moins 20 bars.
